# EUROPEAN PATENT SPECIFICATION

(11) **EP 4 183 351 B1**
(45) Date of publication and mention of the grant of the patent: **28.10.2026**
(21) Application number: 22198088.1
(22) Date of filing: 27.09.2022
(51) Int. Cl.: A61B 17/15, A61B 17/16, A61B 17/17, A61B 17/88, A61B 17/84, A61B 17/80, A61B 17/72, A61B 17/56, A61B 17/66, A61B 17/68

(54) **APPARATUS FOR MINIMALLY INVASIVE BUNION SURGERY**
VORRICHTUNG FÜR MINIMAL INVASIVE BALLENZEHENCHIRURGIE
APPAREIL POUR CHIRURGIE MINI-INVASIVE DE L'OIGNON

(30) Priority: 18.11.2021 US 202163264246 P; 20.09.2022 US 202217933640
(43) Date of publication of application: 24.05.2023
(73) Proprietor: Wright Medical Technology, Inc., Memphis, TN 38117 (US)
(72) Inventor: WOODARD, Joseph Ryan, Memphis, 38117 (US); McGEE, Johnny, Halls, 38040 (US)
(74) Representative: Zimmermann & Partner Patentanwälte mbB

(56) References cited:
- WO-A1-2020/180598
- CN-U- 209 499 835
- US-A1- 2013 190 765
- US-A1- 2021 244 443

## Description

### TECHNICAL FIELD

The disclosed subject matter relates generally a guide system and method to aid an orthopedic surgeon in translating the distal bone fragment and inserting a k-wire into the bone.

### BACKGROUND

Minimally invasive bunion surgery, also known as percutaneous bunionectomy, is a surgical procedure that seeks to correct a bunion deformity by utilizing a small incision. Hallux Valgus is a deformity of the big toe joint wherein a deviation of the big toe towards the lesser toes results in a prominent bump, or bunion.

During a minimally invasive bunion procedure, a surgeon will create an osteotomy in the first metatarsal and then translate a portion of the metatarsal longitudinally prior to installing fixation e.g., Kirschner wire ("k-wires"). Prior art methods require one instrument to be used for translation of the distal bone fragment and another to be used for k-wire targeting. Maintaining adequate translation while applying and using the targeting guide can be cumbersome and represents a challenge to the surgeon.

During any surgery, and minimally invasive surgery in particular, surgeons utilize K-wires to both stabilize the position of bones and to serve as guide wires for screw insertion. It is not uncommon for surgeons to require multiple k-wire insertions before they place the guide wire into the desired position. However, repeated k-wire insertions require time, increase frustration during the procedure, and represent micro-injuries to the bone that the body must heal in addition to the surgical correction.

Current guides/jigs for use in MIS bunion surgery require the surgeon to continuously hold the translation instrument in place in order to maintain the desired translation while targeting the fixation wires. One example, US Patent Application Publication 2020/0060698 entitled SCREW TARGETING GUIDE SYSTEM AND METHOD describes a handheld targeting guide for MIS bunion surgery. The current subject matter presents a jig that combines both features into one instrument and independently maintains the desired translation while the surgeon places the fixation wires.

Document WO 2020/180598 A1 relates to an apparatus and methods for correcting bunion deformities.

Document US 2013/190765 A1 relates to an alignment tool.

Document US 2021/244443 A1 relates to systems, methods, and apparatuses for use in compression/distraction, displacement, and fixation of a calcaneal osteotomy and/or a distal bunion osteotomy.

Reducing the number of instruments, maintaining the desired translation, and enabling the surgeon's hands to focus on k-wire insertion would be beneficial to both the surgeon and patient. Thus, given the deficiencies in the prior art, there remains a need for an improved apparatus to aid in MIS bunion surgery.

### SUMMARY

The embodiments described herein are directed to a system, for bunion correction utilizing a guide for translating the metatarsal head with respect to the metatarsal shaft subsequent an osteotomy and guiding a k-wire utilized in the fixation of the head and shaft in the relative translated position. In addition to or instead of the advantages presented herein, persons of ordinary skill in the art would recognize and appreciate other advantages as well.

In some embodiments, a system for performing a percutaneous bunionectomy is provided. The system including a jig having a body, a shaft contact pad connected to the jig body and conformed to the soft tissue proximate the metatarsal shaft, an arm extending in a longitudinal direction from the jig body, a metatarsal head contact pad connected to the arm and configured to conform to the soft tissue proximate the metatarsal head; a track rigidly connected to the second arm end and movably connected to the jig body and a circular track cooperating with an engagement mechanism of the track, such that a rotation of the track drive results in a lateral movement of the track. The system further includes an alignment guide arm connected to and extending longitudinally from the jig body and a plurality of positioning k-wires, attaching each of the metatarsal head contact pad and the metatarsal shaft contact pad through a multi-dimensional fixation hole to the metatarsal bone. A guide wire is also provided in the system for insertion through the displaced shaft and head of the metatarsal to assist in the subsequent insertion of a fixation screw.

Further disclosed but not claimed is a method the method including the steps of creating an incision in the soft tissue of the foot, positioning a jig upon the soft tissue surrounding the metatarsal bone; securing the jig to the metatarsal bone and, via the incision cutting the metatarsal bone, creating a first portion of the metatarsal and a second portion of the metatarsal. The method further includes translating the jig track to laterally displace the second portion with respect to the first portion; adjusting the visual alignment guide to a desired trajectory; and inserting a fixation k- wire as a screw guide through the first and second portions of the metatarsal such that a fixation screw may permanently fix the bone portions in the laterally displaced position. In the method, the jig is subsequently removed from the metatarsal and the incision is closed.

In yet another embodiment, a translation and insertion jig for insertion of a k-wire is presented. The jig having a body, a shaft contact pad connected to the jig body and conformed to the soft tissue proximate metatarsal shaft, an arm extending in a longitudinal direction from the jig body, a metatarsal head contact pad connected to the arm and configured to conform to the soft tissue proximate the metatarsal head; a track rigidly connected to the second arm end and movably connected to the jig body and a track drive cooperating with an engagement mechanism of the track, such that a rotation of the track drive results in a lateral movement of the track. The system further includes a lock configured to selectively restrain the track from translation relative to the jig body and an alignment guide arm connected to and extending longitudinally from the jig body.

Further disclosed herein is a system for performing a percutaneous bunionectomy, wherein the system includes a jig, which subsequent to attachment and performance of an osteotomy on the bone, uses a rack and pinion mechanism to translate the separated portion of the bone to a surgeon determined displacement λ, wherein a locking mechanism maintains the desired displacement of the bone portions and allows the surgeon, with use of an attached visual guide, to insert a k-wire through the bone portions to serve as a guide wire for the internal fixation that maintains the displacement during the healing, and wherein the system removes the requirement that the surgeon manually maintain the desired displacement while inserting the guide wire.

### BRIEF DESCRIPTION OF THE DRAWINGS

The features and advantages of the present disclosures will be more fully disclosed in, or rendered obvious by, the following detailed descriptions of example embodiments. The detailed descriptions of the example embodiments are to be considered together with the accompanying drawings wherein like numbers refer to like parts and further wherein:
FIG. 1 is an isometric illustration of a translation and insertion jig in accordance with some embodiments of the disclosed subject matter;
FIG. 2 illustrates the translation and insertion jig positioned on the soft tissue near the metatarsal bone of the foot in accordance with some embodiments of the disclosed subject matter;
FIG. 3 illustrates the translation and insertion jig secured to the metatarsal bone via k-wires in accordance with some embodiments of the disclosed subject matter;
FIG. 4 illustrates the translation and insertion jig attached to the metatarsal bone subsequent an osteotomy of the metatarsal bone and prior to the lateral displacement of the metatarsal bone head via the jig in accordance with some embodiments of the disclosed subject matter;
FIG. 5 illustrates the translation and insertion jig attached to the metatarsal bone subsequent to the lateral displacement of the metatarsal bone head via the jig in accordance with some embodiments of the disclosed subject matter;
FIGs. 6A and 6B illustrate in a lateral and A-P view, respectively, the insertion of a guide wire into the metatarsal shaft and laterally displaced head via a visual guide on the translation and insertion jig in accordance with some embodiments of the disclosed subject matter;
FIG. 7 illustrates the track drive and released lock in accordance with an embodiment of the disclosed subject matter; and
FIG. 8 is a flow chart of a method of performing MIS bunion surgery with the translation and insertion jig in accordance with embodiments of the disclosed subject matter.

### DETAILED DESCRIPTION

The description of the preferred embodiments is intended to be read in connection with the accompanying drawings, which are to be considered part of the entire written description of these disclosures. While the present disclosure is susceptible to various modifications and alternative forms, specific embodiments are shown by way of example in the drawings and will be described in detail herein. The objectives and advantages of the claimed subject matter will become more apparent from the following detailed description of these exemplary embodiments in connection with the accompanying drawings.

It should be understood, however, that the present disclosure is not intended to be limited to the particular forms disclosed. Rather, the present disclosure covers all modifications, and alternatives that fall within the scope of these exemplary embodiments. The terms "couple," "coupled," "operatively coupled," "operatively connected," and the like should be broadly understood to refer to connecting devices or components together either mechanically, or otherwise, such that the connection allows the pertinent devices or components to operate with each other as intended by virtue of that relationship.

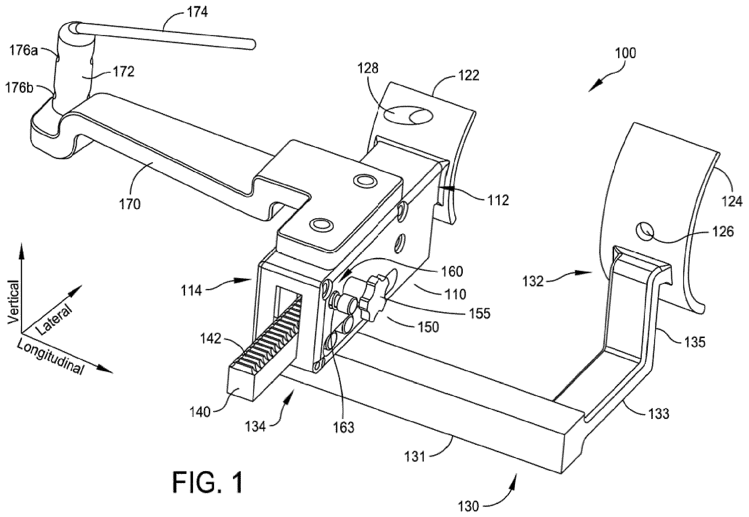

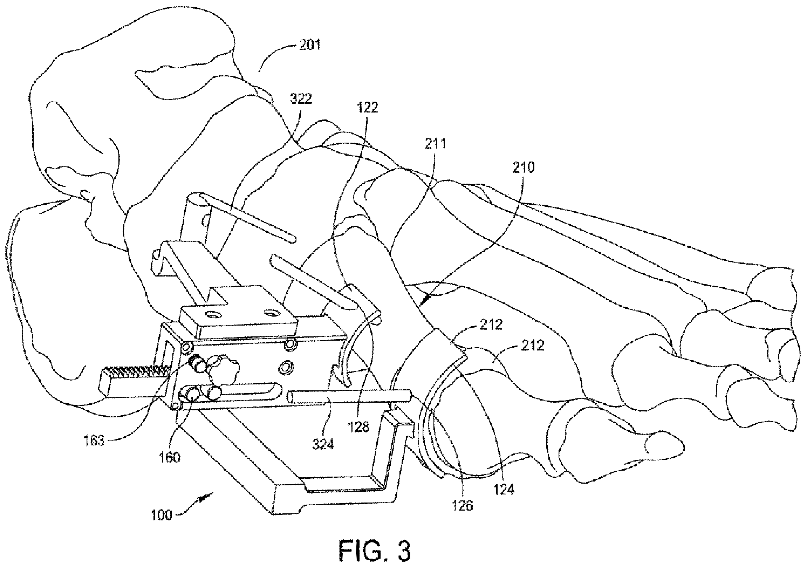

Fig. 1 illustrates a translation and insertion jig 100 for minimally invasive bunion surgery. The jig includes a body 110 which extends from its first end 112 configured to face the bone to a second end 114. The jig 100 includes a metatarsal shaft contact pad 122 connected to the first end 112 of the jig body 110. The shaft contact pad 122 is preferably conformed to the portion of soft tissue (e.g., skin, muscle, fat, fibrous tissue, blood vessels, or other supporting tissue of the body) covering the foot proximate to the metatarsal shaft to which it is intended to engage (e.g. concave). The contour of the shaft contact pad 122 may be conformed during surgery to match the soft tissue surrounding the bone, or pre-contoured. The shaft contact pad 122 may also be selectable by the surgeon from a plurality of different sizes and shapes, such that the jig 100 may have a set of interchangeable contact pads, or pads that attach to the first end 112. The shaft contact pad 122 along with the head contact pad 124 discussed later, include at least one k-wire insertion hole 126, 128 that allows multi-dimensional fixation (i.e., restraint of relative movement in the vertical, lateral and longitudinal directions) of the pads 122, 124 to the respective shaft and head of the metatarsal bone (as shown in Fig. 3).

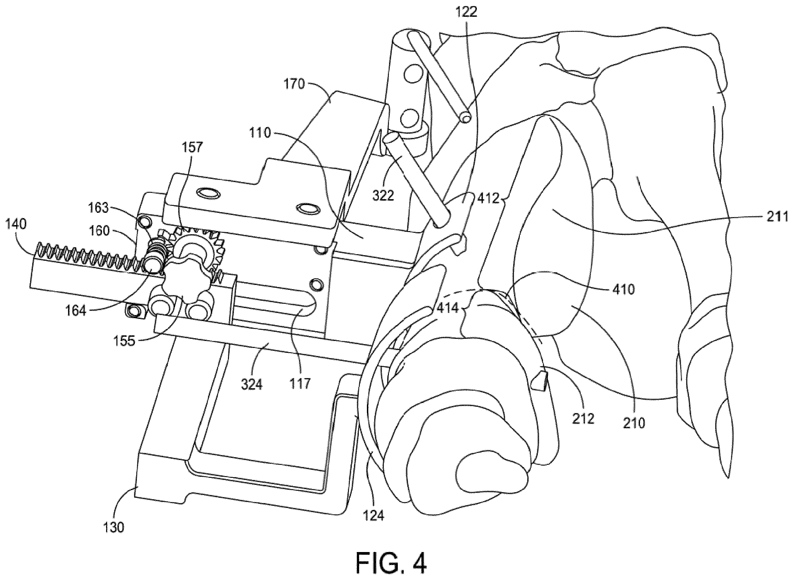

Within or upon the jig body 110 is a linear track 140 extending in the lateral direction, the track 140 rigidly connected to the second end 134 of the extending arm 130 and movably connected to the jig body 110. The linear track 140 includes an engagement mechanism, which, shown in Figs 1-7, is the rack of a rack and pinion mechanism which includes a toothed surface 142. The track 140 alternatively may have a threaded surface such as in a lead screw mechanism. The track 140 is retained to the jig body to allow only lateral movement. Fig. 4 illustrates a groove 117 in the jig body 110 that retains the track 140 in one embodiment, of course other known retention methods are also envisioned. The track 140 may be incremented to enable the surgeon to ascertain the amount of lateral translation and may be further indexed to move one increment (click) at a time, via indentations and ball stops (not shown), or other indexing mechanism known in the art.

The jig 100 has a longitudinal extending arm 130, a second end 134 of the arm 130 attached to a track 140. As with the shaft contact pad 122 and the head contact pad 124 discussed later, the extending arm 130 may be contourable, pre-contoured, or modular such that different sizes and shapes may be selected by the surgeon to be attached directly or indirectly to the track 140. On the other end 132 of the extending arm 130 is a second contact pad 124 for the engagement of the soft tissue surrounding the metatarsal head proximate the pad 124. The contour of the head contact pad 124 may also be conformed during surgery to a shape that matches the soft tissue proximate the bone (e.g. concave) or pre-contoured. The head contact pad 124 may also be selectable by the surgeon from a plurality of different sizes and shapes, such that the jig 100 may have a set of interchangeable contact pads, or pads inserts that attach to the first end 132 of the arm 130. The arm 130 may include a longitudinal section 131, a lateral section 133 and a vertical section 135 to position the head contact pad 124. The longitudinal section 131 and the vertical section 135 may be adjustable by the surgeon to adjust the position of the head contact pad 124, or alternatively may be selectable from a plurality of sizes and shapes in the same manner discussed above.

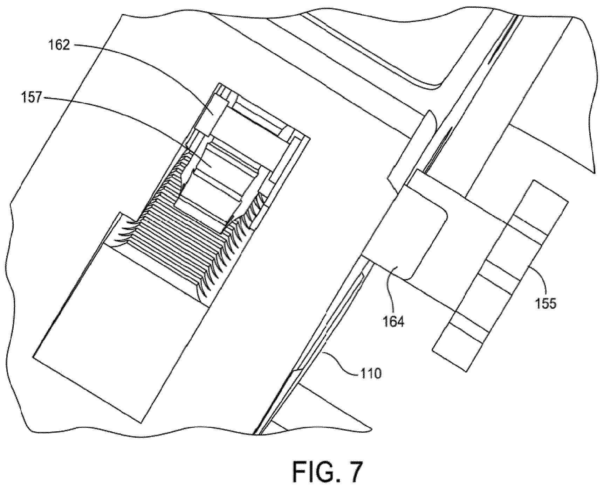

A track drive 150 (*see* also, Figs. 4, 7), including a manual knob 155, dial, or wheel is rotatably connected to the jig body 110, at least a portion of the knob or dial wheel extending outside the jig body 110 to be accessible for manual engagement. The track drive cooperating with the engagement mechanism of the track 140. As shown in Figs. 1 and 7, the track drive 150 includes a circular gear with intermeshing teeth 157 that functions as a pinion in a rack and pinion mechanism, the rack and pinion may also engage frictionally with high friction surfaces on the pinion 150 and cooperating track 140. Alternatively, the track drive may be a worm drive, an internally threaded wheel of a lead screw mechanism, or a ball thread drive, each of which translates rotation of the knob, dial or wheel into linear lateral advancement of the track 140 (i.e., rotation of the track drive moves the track 140 and arm 130 laterally. Each increment of rotation of the circular drive (pinion) or length of track (rack) may be biased into position with known methods such as stop points achieved using teeth or ball plungers so that the track clicks into a plurality of positions laterally spaced from the jig body. Additionally, each increment may be of a predetermined measurement such as a mm or fraction thereof.

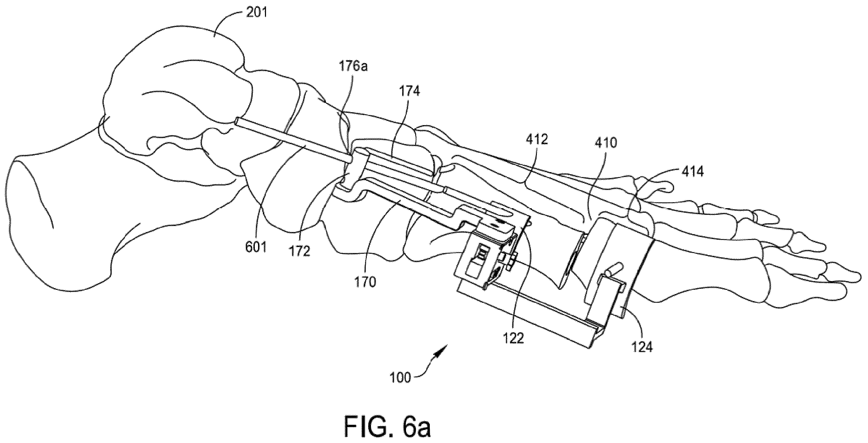

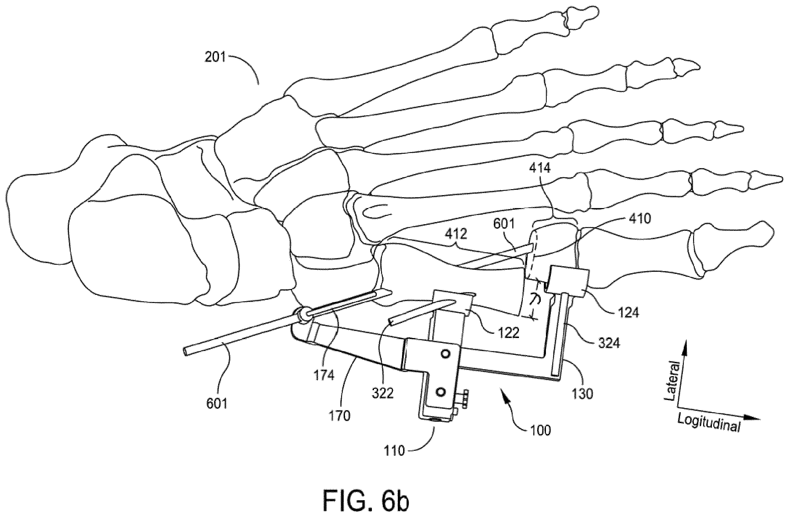

A track lock 160 is attached to the jig body 110 configured to selectively restrain the track from translation relative to the jig body 110. The track lock 160 when engaged prevents the track 140 from retracting laterally and in some embodiments prevents any lateral translation irrespective of direction. The track lock 160 may take the form of a ratchet, allowing the track to advance but not retract, an interlock 162 (as shown in Fig. 7) in which the lock 160 does not allow any translation. The track lock 160 is preferably biased in a locking position, freeing the surgeon from holding the jig to maintain the bone displacement. The track lock 160 may directly engage the track 140 to prevent its translation, or may engage the track drive 150 preventing it from rotating and thus restrict translating. The track lock 160 may alternatively be a threaded restraint screw (not shown) tightened to frictionally engage either the track 140 or drive 150, and loosened to release the same, or vice versa. The track lock 160, shown in Figs. 3 and 6, is biased in the locked position by a spring 163 and released by pressing button 164 removing the interlock 162 from the pinion gears 157, as shown in Fig. 7. With respect to alternative embodiments in which the track drive 150 is a lead screw or worm drive, the mechanical configuration largely limits the need for a track lock 160 since the track 140 cannot drive the track drive 150, thus locking the track 140 in place.

An alignment/targeting guide arm 170 is removably attached to the jig body 110 and extends longitudinally from the jig body 110. A post 172 extends vertically from an end of the alignment guide arm, and visual alignment rod 174 is attached to the post which is selectively rotatable about a vertical axis with respect to the alignment guide arm 170 for establishing the desired trajectory in the A-P plane. Although not shown a second visual alignment rod which is selectively rotatable about a horizontal axis with respect to the alignment guide arm may also be beneficial in establishing the desired trajectory in the lateral plane. The post 172 may feature one or more holes 176a,b aligned with the alignment rod 174. These holes 176a,b may be beneficially sized to accept 3.0 mm and/or 4.0 mm MICA Screw k-wires, for insertion into the metatarsal portions using the jig 100. The visual alignment rod 174 may be created via hole in the vertical post 172 through which a k-wire is inserted to serve as the visual alignment rod 174 when fluoroscopy is used. The alignment guide 170 may also incorporate sleeves through which k-wires are inserted to ensure the wires enter the bone at the proper trajectory.

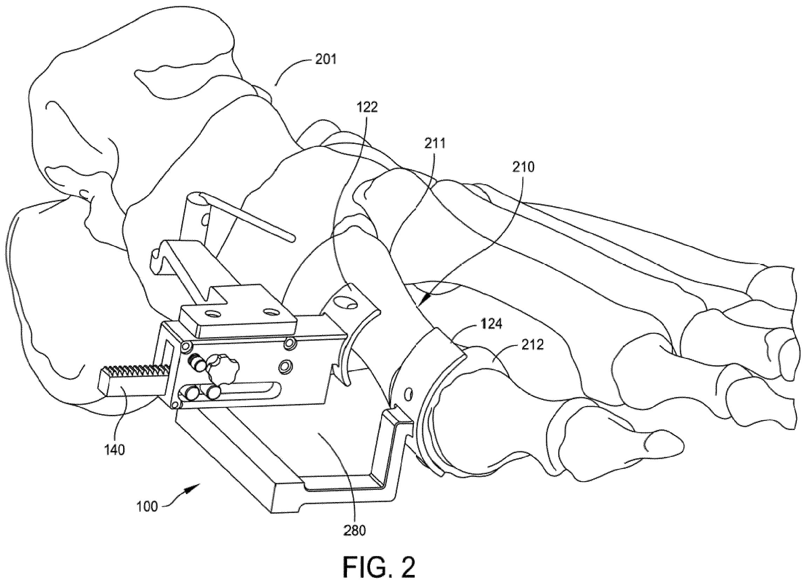

Fig. 2 illustrates the jig 100 positioned on the soft tissue (not shown) proximate the metatarsal 210 of a foot 201. The shaft contact pad 122 is positioned upon the soft tissue covering the metatarsal shaft 211 and the head contact pad 124 is positioned upon the soft tissue covering the metatarsal head 212. As shown in Fig. 1, the track 140 and arm 130 are in the retracted or initial position. The extending arm 130 may be configured with respect to the anticipated incision location so as not to interfere with the osteotomy instruments access to the metatarsal bone 210 via the incision. As shown in Fig. 2, the shape of the extending arm 130 leaves an access area 280 to the metatarsal 210 between 270 and 360 degrees.

Fig. 3 illustrates the attachment of the jig 100 to the metatarsal bone 210 via k-wires 324 and 322. The respective k-wires 322 and 324 are inserted through holes 126 and 128 in contact pads 124 and 122 respectively through the soft tissue and into the shaft 211 and head 212 of the metatarsal 210. The holes 126 and 128 are preferably oriented to direct the k-wires into the bone to ensure multi-dimensional fixation of each of the contact pads 122 and 124 to the shaft 211 and head 212 of the metatarsal bone 210. The multi-dimensional fixation of each portion of the metatarsal bone 210 is desirable to ensure the jig 100 remains secured to the respective bone portions subsequent the osteotomy.

In Fig. 4, the jig 100 positioned on the metatarsal bone 210 is shown subsequent to the osteotomy 410. As a result of the osteotomy 410, the metatarsal 210 is divided into a shaft portion 412, including the shaft 211, being attached to the shaft contact pad 122 and a head portion 414 including the head 212 being attached to the head contact pad 124. The track 140 and extending arm 130 are shown in a retracted position or initial position prior to the translation of the head portion 414 with respect to the shaft portion 412 via the jig 100.

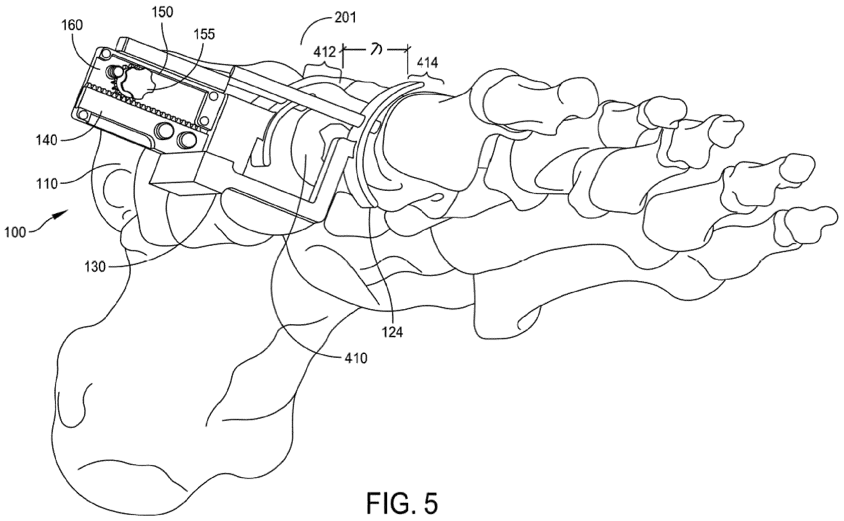

Fig. 5 illustrates the jig 100 with the track 140, arm 130 and the head contact pad 124 extended to the desired location selected by the surgeon. As can be seen in Fig. 5, at the osteotomy 410, the head portion 414 has been translated laterally from the shaft portion 412 by λ. The track 140 is configured to laterally translate from a first position as shown in Figs. 2, 3 and 4 to a fully translated position including any intermediate surgeon selectable position as shown in Fig. 5 and 6B. The track 140 is restrained to a predetermined path, typically a straight line, however, it may be beneficial in some circumstances for the path to be curved or not linear.

Turning to Figs. 6A and 6B, the insertion of the guide wire 601 through the shaft portion 412 and head portion 414 of the metatarsal 210 is shown. With the translation λ established and maintained via the jig 100, the k-wire 601 is placed through the k-wire hole (176a,b) in the post 172 and inserted into the metatarsal 210 following the trajectory established by visual guide rod 174. A screw may then be guided by the guide wire 601 into the shaft portion 412 and head portion 414 of the metatarsal to fix the translation λ throughout the healing period. Because of the lock 160 or the inherent nature of the track drive 150, the displacement λ is maintained by the jig 100 without the necessity of surgeon's attention or hands, which are freed for the insertion of the guide wire 601 and ultimately the fixation screw (not shown).

The visual guide 174 enables the surgeon to visualize the relative position of the surgical k-wire 601 within both portions of the bone 210. FIG. 6B illustrates an anterior posterior (A-P) view, i.e. looking down upon the foot 201. The visual guide rod 174 is coincident with the surgical k-wire 601 when the k-wire 601 is following the prescribed trajectory in the A-P view as shown in Fig. 6B. If a second visual guide is provided, it would be coincident with the surgical k-wire 601 when the k-wire 601 is following the prescribed trajectory in the lateral view, generally shown in Fig. 6A. Thus the location and movement of the inserted k-wire 601 in the A-P view may be represented by the visual guide rod 174, additionally, the visual guide rod 174 may also be rotatable with respect to the horizon and thus act as an insertion guide in the lateral plane (i.e. the plane normal to the lateral direction), though it requires the surgeon to account for the vertical offset in the lateral view. Likewise, in FIG 6A, the second guide rod (not shown) may govern movement of the inserted k-wire 601 in the lateral plane. Thus between the top and side views of FIGS. 6A and 6B respectively, the location and trajectory of the inserted k-wire 601 may be represented in three dimensions and thus monitored and controlled using one or two visual guide rods.

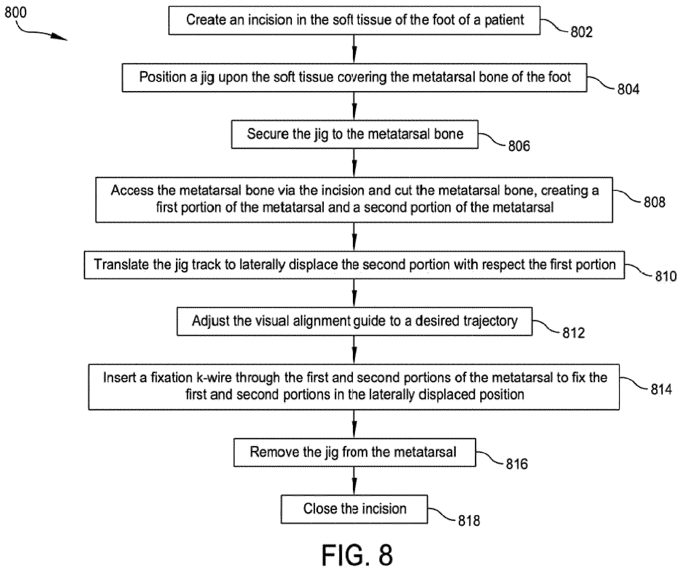

Fig. 8 illustrates a method 800 for performing a percutaneous bunionectomy using the jig 100. First, as is common in a conventional percutaneous bunionectomy, an incision in the soft tissue of the foot 201 of a patient is created by the surgeon as shown in Block 802. The jig 100 is positioned upon the soft tissue (not shown) proximate the metatarsal bone 210 of the foot 201 as shown in Block 804 (see also Fig 2 which illustrates the jig 100 on the soft tissue proximate the metatarsal 210). As properly positioned, the shaft contact pad 122 and a head contact pad 124 of the jig 100 are proximate the shaft 211 and head 212 respectively of the metatarsal bone 210.

In Block 806, the jig 100 is secured to the metatarsal bone 210 as illustrated in Fig. 3. In Fig. 3, the jig 100 is secured to the bone via k-wires 322 and 324 inserted through the contact pads, soft tissue and into the bone, however it is envisioned other fastening devices (i.e. screws, pins, clamps etc.) may also have beneficial applications.

In Block 808, the metatarsal bone is accessed through the incision and is cut creating first and second portions 412 and 414 of the metatarsal 210, the result of the osteotomy 410 is illustrated in Fig. 4. The jig track 140 is then translated by manually rotating knob 155 of the track drive 150 such that the desired displacement λ is achieved between the two portions as shown in Block 810. Fig. 5 illustrate the track 140, arm 130, head contact pad 124 and head portion 414 of the metatarsal with the desired displacement λ. As noted above, the locking mechanism 160 or the mechanical configuration of the track drive 150 prevents the track 140 from retracting, thus in some embodiments, the locking mechanism must be activated subsequent translation, or in the case where the locking mechanism 160 is biased in a locking position, the locking mechanism would be released to allow the translation of the track 140.

As shown in Block 812, the visual guide rod 174 of the alignment guide 170 is adjusted to the desired trajectory. Fig. 6B illustrates the visual guide rod 174 aligned along the surgeon selected trajectory. In Block 814, a guide wire is inserted through the first and second portions 412 and 414 of the metatarsal 210 to fix the first and second portions in the laterally displaced position. Fig. 6A illustrates the k-wire 601 inserted through the shaft portion 412 and the head portion 414 when they are displaced by λ. The surgical k-wire 601 is positioned through one of the guide holes 176a, 176b and into the soft tissue of the foot 201. The k-wire 601 is advanced while aligning it with the visual guide rod 174 in the A-P plane, and if a second guide rod is provided, then aligning the k-wire 601 with the other guide rod in the lateral plane. The surgeon may check the position in both A-P and lateral planes to determine trajectory of surgical k-wire and adjust as needed. These latter steps typically require use of fluoroscopy to confirm intended trajectory has been achieved. The surgical k-wire 601 is advanced into the bone while maintaining the desired trajectory in the lateral and A-P planes with reference to the respective visual guides. The surgical k-wire may be advanced incrementally and its position and orientation again verified via the visual guide as it is advanced. As noted, a fluoroscope is preferably used to visualize the bone and the surgical k-wire with reference to the visual guide. Thus, it is preferable that the visual guide rod 174 is not X-ray permeable, so it will appear on the fluoroscope. As shown in Block 816, the jig 100 is removed from the metatarsal 210. Subsequent removal of the jig 100, the surgeon may use a depth gage placed over the guide k-wire 601 to measure screw length. The surgeon then places a drill over the k-wire 601 with a cannulated drill, then inserts a fixation screw over the k-wire to properly fixate the osteotomy. The guide wire 601 is then removed. A second screw may also be placed lateral to this screw depending on patient anatomy and needs. The incision is closed in Block 818.

The visual guide 170, as discussed above, may have one or more guide rods that extend parallel to the surgical k-wire and serve as representations of the surgical k-wire within the respective planes, to assist in its proper insertion.

Another aspect of the disclosed subject matter is that the visual guides may be any narrow elongated straight rod, strip or bar that achieve the desired goal of visually referencing the position of the surgical k-wire being inserted. Examples may be rods formed from plastic, ceramic, metal or organic material, or composites thereof. For materials that are x-ray permeable, non-permeable material may be added to enable the guide to be visible under a fluoroscope.

While the jig 100 shown in Figs 1-7, is configured for the left foot, it is equally envisioned that the jig could be likewise configured for the right foot, or even configured for universal use on both the left and right feet with minor adjustments as understood by one skilled in the art. Additionally, although the disclosed subject matter is described with respect to a rack and pinion system (i.e. the track is the rack and the drive is the pinion) other configurations are also envisioned, such as a worm drive for translating the track, ball-screw drive system, or a lead screw system. Additionally, while the lock is described with respect an interlock key, other configurations, such as a frictional lock or ratcheting lock are also envisioned.

The method shown in Fig. 8, describes the positioning and securing of the jig 100 prior to the creation of the incision, and/or the osteotomy. However, one or both of the incision and the osteotomy may be performed prior to the positioning and/or securing of the jig 100. In the case where the osteotomy is preformed prior to the securing of the jig 100, the jig 100 need not account for access 280 to the bone 210, other than for the guide wire 601 and subsequent fixation screw.

Another aspect of the disclosed subject matter is that although the jig 100 is described in view of minimally invasive bunion surgery, it may also be useful in other osteotomies in which portions of a bisected bone are displaced relative to one another, as may be appreciated by those skilled in the art.

Although the methods described above are with reference to the illustrated flowchart, it will be appreciated that many other ways of performing the acts associated with the methods can be used. For example, the order of some operations may be changed, and some of the operations described may be optional.

The foregoing is provided for purposes of illustrating, explaining, and describing embodiments of these disclosures. Modifications and adaptations to these embodiments will be apparent to those skilled in the art and may be made without departing from the scope of these disclosures.

## Claims

1. An adjustable jig (100) for a minimally invasive bunionectomy surgery, during which a surgeon creates an osteotomy in a first metatarsal and then translates a portion of the metatarsal longitudinally prior to installing fixation, comprising:
a jig body (110), the jig body (110) extending in a lateral direction between a first body end (112) and an oppositely disposed second body end (114);
an arm (130) extending in a longitudinal direction from the jig body (110), the arm having a first arm end (132) and a second arm end (143);
a linear track (140) extending in the lateral direction, the track (140) rigidly connected to the second arm end (143) and movably connected to the jig body (110), the track (140) including an engagement mechanism;
a circular track drive (150) rotatably connected to the jig body (110), the circular track drive (150) cooperating with the engagement mechanism of the track (140), such that a rotation of the circular track drive (150) results in a lateral movement of the track (140); and
a lock (160) connected to the jig body (110) and configured to selectively restrain the track (140) from translation relative to the jig body (110);
**characterised in that** the adjustable jig comprises further:
a metatarsal shaft contact pad, the metatarsal shaft contact pad connected to the first body end, and
configured to conform to a portion of soft tissue proximate the metatarsal shaft of the metatarsal; and
a metatarsal head contact pad, the metatarsal head contact pad connected to the first arm end, and
configured to conform to a portion of soft tissue proximate the metatarsal head of the metatarsal.

2. The adjustable jig (100) of Claim 1, further comprising an alignment guide arm, the alignment guide arm connected to and extending longitudinally from the jig body (110).

3. The adjustable jig (100) of any of claims 1-2, wherein the engagement mechanism comprises a plurality of teeth and track drive (150) comprises a plurality of intermeshing teeth.

4. The adjustable jig (100) of any of claims 1-3, wherein the engagement mechanism comprises a high friction surface.

5. The adjustable jig (100) of any of claims 1-4, wherein the lock is configured to selectively engage the engagement mechanism of the track (140) or the circular track drive (150) to restrain the track (140).

6. The adjustable jig (100) of Claim 5, wherein the lock is biased to restrain the track (140).

7. The adjustable jig (100) of any of claims 1-6, wherein the metatarsal head contact pad (124) and the metatarsal shaft contact pad (122) are concave.

8. The adjustable jig (100) of any of claims 1-7, wherein each of the metatarsal head contact pad (124) and the metatarsal shaft contact pad (122), comprise a k-wire multi-dimensional fixation hole.

9. The adjustable jig (100) of Claim 6, further comprising a post extending vertically from an end of the alignment guide arm, and a visual alignment rod attached to the post and selectively rotatable about a vertical axis with respect to the alignment guide arm.

10. The adjustable jig (100) of any of claims 1-9, further comprising a second visual alignment rod selectively rotatable about a horizontal axis with respect to the alignment guide arm.

11. The adjustable jig (100) of any of claims 1-10, wherein the circular track drive (150) engages with the engagement mechanism within the jig body (110), the circular track drive (150) further comprising a drive knob (155), a portion of the knob external to the jig body (110).

12. The adjustable jig (100) of any of claims 1-11, wherein the linear track (140) is a rack and the circular track drive (150) is a pinion, and/or wherein the jig body (110) further restraining the linear track (140) longitudinally to a predetermined path.

13. The adjustable jig (100) of Claim 9, wherein the post defines a hole for receiving a guide wire.

14. A system for performing a percutaneous bunionectomy, during which a surgeon creates an osteotomy in a first metatarsal and then translates a portion of the metatarsal longitudinally prior to installing fixation, comprising:
the jig (100) of claim 1, and an alignment guide arm (170), the alignment guide arm (170) connected to and extending longitudinally from the jig body (110);
a plurality of positioning k-wires, wherein each of the metatarsal head contact pad (124) and the metatarsal shaft contact pad (122), comprise a multi-dimensional fixation hole (126, 128) configured to receive one of the plurality of positioning k-wires;
and at least one fixation guide k-wire.

## Patentansprüche

1. Einstellbare Spannvorrichtung (100) für eine minimalinvasive Bunionektomie-Operation, im Laufe derer ein Chirurg eine Osteotomie in einem ersten Mittelfußknochen erzeugt und dann vor dem Installieren einer Fixierung einen Anteil des Mittelfußknochens in Längsrichtung verschiebt, umfassend:
einen Spannvorrichtungskörper (110), wobei sich der Spannvorrichtungskörper (110) in einer Querrichtung zwischen einem ersten Körperende (112) und einem gegenüberliegend angeordneten zweiten Körperende (114) erstreckt;
einen Arm (130), der sich in einer Längsrichtung von dem Spannvorrichtungskörper (110) erstreckt, wobei der Arm ein erstes Armende (132) und ein zweites Armende (143) aufweist;
eine lineare Schiene (140), die sich in der Querrichtung erstreckt, wobei die Schiene (140) starr mit dem zweiten Armende (143) verbunden und beweglich mit dem Spannvorrichtungskörper (110) verbunden ist, wobei die Schiene (140) einen Eingriffsmechanismus umfasst;
einen kreisförmigen Schienenantrieb (150), der drehbar mit dem Spannvorrichtungskörper (110) verbunden ist, wobei der kreisförmige Schienenantrieb (150) mit dem Eingriffsmechanismus der Schiene (140) derart zusammenwirkt, dass eine Drehung des kreisförmigen Schienenantriebs (150) in einer seitlichen Bewegung der Schiene (140) resultiert; und
eine Verriegelung (160), die mit dem Spannvorrichtungskörper (110) verbunden und konfiguriert ist, um die Schiene (140) von einer Verschiebung relativ zu dem Spannvorrichtungskörper (110) zurückzuhalten;
**dadurch gekennzeichnet, dass** die einstellbare Spannvorrichtung ferner Folgendes umfasst:
ein Mittelfußknochenschaft-Kontaktpolster, wobei das Mittelfußknochenschaft-Kontaktpolster mit dem ersten Körperende verbunden und konfiguriert ist, um sich an einen Anteil von Weichgewebe nahe dem Mittelfußknochenschaft des Mittelfußknochens anzupassen; und ein Mittelfußkopf-Kontaktpolster, wobei das Mittelfußkopf-Kontaktpolster mit dem ersten Armende verbunden ist und
konfiguriert ist, um sich an einen Anteil von Weichgewebe nahe dem Mittelfußkopf des Mittelfußknochens anzupassen.

2. Einstellbare Spannvorrichtung (100) nach Anspruch 1, ferner umfassend einen Ausrichtungsführungsarm, wobei der Ausrichtungsführungsarm mit dem Spannvorrichtungskörper (110) verbunden ist und sich in Längsrichtung von diesem erstreckt.

3. Einstellbare Spannvorrichtung (100) nach einem der Ansprüche 1 bis 2, wobei der Eingriffsmechanismus eine Vielzahl von Zähnen umfasst und der Schienenantrieb (150) eine Vielzahl von ineinandergreifenden Zähnen umfasst.

4. Einstellbare Spannvorrichtung (100) nach einem der Ansprüche 1 bis 3, wobei der Eingriffsmechanismus eine Oberfläche mit hoher Reibung umfasst.

5. Einstellbare Spannvorrichtung (100) nach einem der Ansprüche 1 bis 4, wobei die Verriegelung zum selektiven Eingreifen in den Eingriffsmechanismus der Schiene (140) oder den kreisförmigen Schienenantrieb (150) konfiguriert ist, um die Schiene (140) zurückzuhalten.

6. Einstellbare Spannvorrichtung (100) nach Anspruch 5, wobei die Verriegelung vorgespannt ist, um die Schiene (140) zurückzuhalten.

7. Einstellbare Spannvorrichtung (100) nach einem der Ansprüche 1 bis 6, wobei das Mittelfußkopf-Kontaktpolster (124) und das Mittelfußknochenschaft-Kontaktpolster (122) konkav sind.

8. Einstellbare Spannvorrichtung (100) nach einem der Ansprüche 1 bis 7, wobei jedes von dem Mittelfußkopf-Kontaktpolster (124) und dem Mittelfußknochenschaft-Kontaktpolster (122) ein mehrdimensionales K-Draht-Fixierungsloch umfassen.

9. Einstellbare Spannvorrichtung (100) nach Anspruch 6, ferner umfassend einen Pfosten, der sich vertikal von einem Ende des Ausrichtungsführungsarms erstreckt, und einen visuellen Ausrichtungsstab, der an dem Pfosten angebracht und in Bezug auf den Ausrichtungsführungsarm selektiv um eine vertikale Achse drehbar ist.

10. Einstellbare Spannvorrichtung (100) nach einem der Ansprüche 1 bis 9, ferner umfassend einen zweiten visuellen Ausrichtungsstab, der in Bezug auf den Ausrichtungsführungsarm selektiv um eine horizontale Achse drehbar ist.

11. Einstellbare Spannvorrichtung (100) nach einem der Ansprüche 1 bis 10, wobei der kreisförmige Schienenantrieb (150) in den Eingriffsmechanismus innerhalb des Spannvorrichtungskörpers (110) eingreift, wobei der kreisförmige Schienenantrieb (150) ferner einen Antriebsknopf (155) umfasst, wobei ein Anteil des Knopfes außerhalb des Spannvorrichtungskörpers (110) vorliegt.

12. Einstellbare Spannvorrichtung (100) nach einem der Ansprüche 1 bis 11, wobei die lineare Schiene (140) eine Zahnstange ist und der kreisförmige Schienenantrieb (150) ein Ritzel ist und/oder wobei der Spannvorrichtungskörper (110) ferner die lineare Schiene (140) in Längsrichtung auf einem vorbestimmten Pfad zurückhält.

13. Einstellbare Spannvorrichtung (100) nach Anspruch 9, wobei der Pfosten ein Loch zum Aufnehmen eines Führungsdrahtes definiert.

14. System zum Durchführen einer perkutanen Bunionektomie, während der ein Chirurg eine Osteotomie in einem ersten Mittelfußknochen erzeugt und dann vor dem Installieren einer Fixierung einen Anteil des Mittelfußknochens in Längsrichtung verschiebt, umfassend:
die Spannvorrichtung (100) nach Anspruch 1 und einen Ausrichtungsführungsarm (170), wobei der Ausrichtungsführungsarm (170) mit dem Spannvorrichtungskörper (110) verbunden ist und sich in Längsrichtung von diesem erstreckt;
eine Vielzahl von Positionierungs-K-Drähten, wobei jedes von dem Mittelfußkopf-Kontaktpolster (124) und dem Mittelfußknochenschaft-Kontaktpolster (122) ein mehrdimensionales Fixierungsloch (126, 128) umfassen, das konfiguriert ist, um einen der Vielzahl von Positionierungs-K-Drähten aufzunehmen;
und mindestens einen Fixierungsführungs-K-Draht.

## Revendications

1. Gabarit réglable (100) pour une chirurgie minimalement invasive de l'hallux valgus, pendant laquelle un chirurgien crée une ostéotomie dans un premier métatarsien et ensuite fait translater une partie du métatarsien de manière longitudinale avant de mettre en place une fixation, comprenant :
un corps de gabarit (110), le corps de gabarit (110) s'étendant dans une direction latérale entre une première extrémité de corps (112) et une deuxième extrémité de corps (114) disposée à l'opposé ;
un bras (130) s'étendant dans une direction longitudinale à partir du corps de gabarit (110), le bras ayant une première extrémité de bras (132) et une deuxième extrémité de bras (143) ;
un rail linéaire (140) s'étendant dans la direction latérale, le rail (140) étant rigidement raccordé à la deuxième extrémité de bras (143) et raccordé, de manière mobile, au corps de gabarit (110), le rail (140) comprend un mécanisme de mise en prise ;
un dispositif d'entraînement circulaire de rail (150) raccordé, de manière rotative, au corps de gabarit (110), le dispositif d'entraînement circulaire de rail (150) coopérant avec le mécanisme de mise en prise du rail (140), de sorte qu'une rotation du dispositif d'entraînement circulaire de rail (150) se traduit par un déplacement latéral du rail (140) ; et
un verrou (160) raccordé au corps de gabarit (110) et configuré pour empêcher sélectivement le rail (140) de se déplacer en translation par rapport au corps de gabarit (110) ;
**caractérisé en ce que** le gabarit réglable comprend en outre :
une pastille de contact de corps de métatarsien, la pastille de contact de corps de métatarsien étant raccordée à la première extrémité de corps, et configurée pour se conformer à une partie du tissu mou à proximité du corps de métatarsien du métatarsien ; et
une pastille de contact de tête de métatarsien, la pastille de contact de tête de métatarsien étant raccordée à la première extrémité de bras, et configurée pour se conformer à une partie du tissu mou à proximité de la tête de métatarsien du métatarsien.

2. Gabarit réglable (100) selon la revendication 1, comprenant en outre un bras de guidage d'alignement, le bras de guidage d'alignement étant raccordé à et s'étendant, de manière longitudinale, à partir du corps de gabarit (110).

3. Gabarit réglable (100) selon l'une quelconque des revendications 1 à 2, dans lequel le mécanisme de mise en prise comprend une pluralité de dents et le dispositif d'entraînement de rail (150) comprend une pluralité de dents engrenées entre elles.

4. Gabarit réglable (100) selon l'une quelconque des revendications 1 à 3, dans lequel le mécanisme de mise en prise comprend une surface à haute friction.

5. Gabarit réglable (100) selon l'une quelconque des revendications 1 à 4, dans lequel le verrou est configuré pour mettre, sélectivement, en prise le mécanisme de mise en prise du rail (140) ou du dispositif d'entraînement circulaire de rail (150) pour retenir le rail (140).

6. Gabarit réglable (100) selon la revendication 5, dans lequel le verrou est sollicité pour retenir le rail (140).

7. Gabarit réglable (100) selon l'une quelconque des revendications 1 à 6, dans lequel la pastille de contact de tête de métatarsien (124) et la pastille de contact de corps de métatarsien (122) sont concaves.

8. Gabarit réglable (100) selon l'une quelconque des revendications 1 à 7, dans lequel chacune parmi la pastille de contact de tête de métatarsien (124) et la pastille de contact de corps de métatarsien (122) comprennent un trou de fixation multidimensionnel pour broche de Kirschner.

9. Gabarit réglable (100) selon la revendication 6, comprenant en outre un montant s'étendant verticalement à partir d'une extrémité du bras de guidage d'alignement et une tige d'alignement visuel fixée au montant et pouvant tourner, de manière sélective, autour d'un axe vertical par rapport au bras de guidage d'alignement.

10. Gabarit réglable (100) selon l'une quelconque des revendications 1 à 9, comprenant en outre une deuxième tige d'alignement visuel pouvant tourner, de manière sélective, autour d'un axe horizontal par rapport au bras de guidage d'alignement.

11. Gabarit réglable (100) selon l'une quelconque des revendications 1 à 10, dans lequel le dispositif d'entraînement circulaire de rail (150) se met en prise avec le mécanisme de mise en prise à l'intérieur du corps de gabarit (110), le dispositif d'entraînement circulaire de rail (150) comprenant en outre un bouton de dispositif d'entraînement (155), une partie du boîtier étant à l'extérieur du corps de gabarit (110).

12. Gabarit réglable (100) selon l'une quelconque des revendications 1 à 11, dans lequel le rail linéaire (140) est une crémaillère et le dispositif d'entraînement circulaire de rail (150) est un pignon, et/ou le corps de gabarit (110) contraignant en outre le rail linéaire (140), de manière longitudinale, selon une trajectoire prédéterminée.

13. Gabarit réglable (100) selon la revendication 9, dans lequel le montant définit un trou pour recevoir un fil-guide.

14. Système destiné à réaliser une bunionectomie percutanée, pendant laquelle un chirurgien crée une ostéotomie dans un premier métatarsien et fait ensuite translater une partie du métatarsien, de manière longitudinale, avant de mettre en place une fixation, comprenant :
le gabarit (100) selon la revendication 1 et un bras de guidage d'alignement (170), le bras de guidage d'alignement (170) étant raccordé à et s'étendant, de manière longitudinale, à partir du corps de gabarit (110) ;
une pluralité de broches de Kirschner de positionnement, dans lequel chacune parmi la pastille de contact de tête de métatarsien (124) et la pastille de contact de corps de métatarsien (122), comprend un trou de fixation multidimensionnel (126, 128) configuré pour recevoir l'une de la pluralité de broches de Kirschner de positionnement ;
et au moins une broche de Kirschner de guidage de fixation.
